**Europäisches Patentamt**

**European Patent Office**

**Office européen des brevets**

(11) Publication number: **0 160 703**
**B1**

# EUROPEAN PATENT SPECIFICATION

(45) Date of publication of patent specification: **02.05.90**

(51) Int. Cl.⁵: **A 61 N 2/02**

(21) Application number: **83903311.5**

(22) Date of filing: **26.10.83**

(86) International application number:
**PCT/JP83/00382**

(87) International publication number:
**WO 85/01881 09.05.85 Gazette 85/11**

(54) MAGNETIC FIELD GENERATING THERAPEUTIC APPLIANCE.

(43) Date of publication of application:
**13.11.85 Bulletin 85/46**

(45) Publication of the grant of the patent:
**02.05.90 Bulletin 90/18**

(84) Designated Contracting States:
**DE FR GB NL**

(56) References cited:
**DE-A-2 853 365**
**DE-A-3 246 128**
**FR-A-1 207 360**
**JP-U-57 076 641**
**JP-Y-36 023 089**
**JP-Y-54 015 508**
**US-A-4 266 532**

(73) Proprietor: **NIHONKENKOZOSHINKENKYUKAI CO. LTD.**
**30-30 Najima 1-chome Higashi-ku**
**Fukuoka-shi Fukuoka 813 (JP)**

(72) Inventor: **ONISHI, Teruo**
**2-33, Kotobukicho Toyooka-shi**
**Hyogo 668 (JP)**

(74) Representative: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund Morgan, B.Sc.(Phys.) Robert-Koch-Strasse 1 D-8000 München 22 (DE)**

Courier Press, Leamington Spa, England.

## Description

The present invention relates to a therapeutic apparatus for generating a magnetic field in accordance with the preamble of claim 1. Such an apparatus is disclosed in Japanese Utility Model JP—Y1—36—23089.

The present disclosure has particular reference to a therapeutic apparatus for generating an alternating magnetic field which treats pains, stiffness of the body and other ailments of patients through enhancing blood circulation by continuous application of the alternating magnetic field to the affected portion of the body.

It will be noted that the therapeutic apparatus of the above cited Japanese Utility Model is constituted by a plurality of metal boxes which are connected in series through intermediate link-like connecting members with a permanent magnet being housed inside each metal box. In the known arrangement the metal boxes are not directly connected with each other, so that the permanent magnets are spaced apart from one another.

Other therapeutic apparatus for generating a magnetic field is known and is constituted e.g. by a magnetic generator housed inside a case of box or cylindrical shape with a construction such that a patient or a third person presses the apparatus on the affected portion of the patient's body by hand to apply the alternating magnetic field.

However, this type of apparatus can perform therapy on only a limited part of the body because of the narrow range of the magnetic field that is applicable. Moreover, it is necessary to move the apparatus, particularly when the affected part is wide ranging. In addition, the effect of the magnetic therapy is insufficient because of the low intensity of the magnetic field. Furthermore, the manoeuvrability is poor due to the hand operation that is required, this causes trouble in therapy and the operator becomes fatigued.

Another known medical treatment apparatus is disclosed in FR—A—1 207 360. In this arrangement the apparatus has the form of an elongate rail which is described as being of U-shape, with the free end portions of the limbs of the U being bent inwardly to face one another and form pole pieces. In operation the portion of the body to be treated, for example a forearm, is placed in the rail between the confronting pole pieces. The rail is either made up of one long permanent magnet or a plurality of permanent magnets arranged in series. In one embodiment the rail is made up of a plurality of U shaped sections arranged in series with coils wound around the sections so that the sections are converted into electromagnets.

Reference should also be made to US—A—4 266 532 which describes a magnetic treatment apparatus which is electrically energised by a variable DC source which is chopped to form DC current pulses. In one embodiment two coils are connected together by Velcro (Registered Trademark) strips and can be placed on opposite sides of a limb or body part requiring treatment, with the two coils confronting one another.

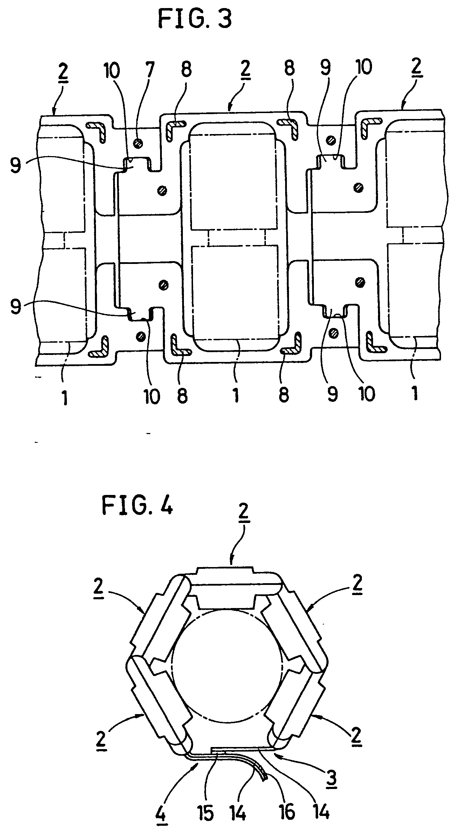

Another apparatus in accordance with the preamble of claim 1 is known from DE—A—32 46 128 in which the magnetic field generators comprise permanent magnets. In Fig. 3 of this citation the carriers for the permanent magnets which can be rigid or flexible are jointedly connected together in one direction and thus form a band which can be slung around the body as a bracelet, a neck band or a foot band, or which can be slung around the body in the form of a belt. The aim of this arrangement is to enlarge the useful surface of the individual magnets without magnetism from the other pole arriving at the useful surface.

Thus the conventional apparatuses have disadvantages as mentioned above.

The object underlying the present invention is to provide a novel therapeutic apparatus for generating a magnetic field which excels in its therapeutic effect and has a construction which is easy to manoeuvre and fix to the patient's body, and which also exerts a therapeutic action through vibration and heating.

In order to satisfy this object the present invention provides a therapeutic apparatus having the characterising features of claim 1.

Thus a plural number of cases each housing a magnetic field generator are respectively pivotally connected directly to one another in a freely rotatable fashion. The connecting members at the two end cases can be wound round and fixed to any part of the patient's body such as the neck, waist, arm, leg, or the like. Most important is the fact that fluctuating magnetic fields are generated individually from each magnetic field generator. These magnetic fields can be made to influence the affected part intensively over a wide range while surrounding it. Moreover, the cases are caused to vibrate and the vibration exerts a massaging effect on the body. In addition a warming effect is provided by the generation of Joule heat. Accordingly an excellent therapeutic effect is obtained in comparison with conventional apparatus and, moreover, no special operation like moving the apparatus is required, meaning that the manoeuvrability is enhanced.

The invention will now be explained in more detail with reference to the accompanying drawings which illustrate the best known form of an apparatus embodying the invention. In the drawings:

Fig. 1 is a front view showing an entire configuration of a therapeutic apparatus in accordance with the present invention for generating a magnetic field,

Fig. 2 is an enlarged cross-sectional view wherein cases in a coupled state are cut in the horizontal direction,

Fig. 3 is an enlarged cross-sectional view wherein cases in a coupled state are cut in the vertical direction,

Fig. 4 is a plan view showing a state of use of the therapeutic apparatus for generating a magnetic field,

Fig. 5 is a front view showing a configuration of a magnetic field generator, and

Fig. 6 is a block diagram showing the electric wiring of the therapeutic apparatus for generating a magnetic field.

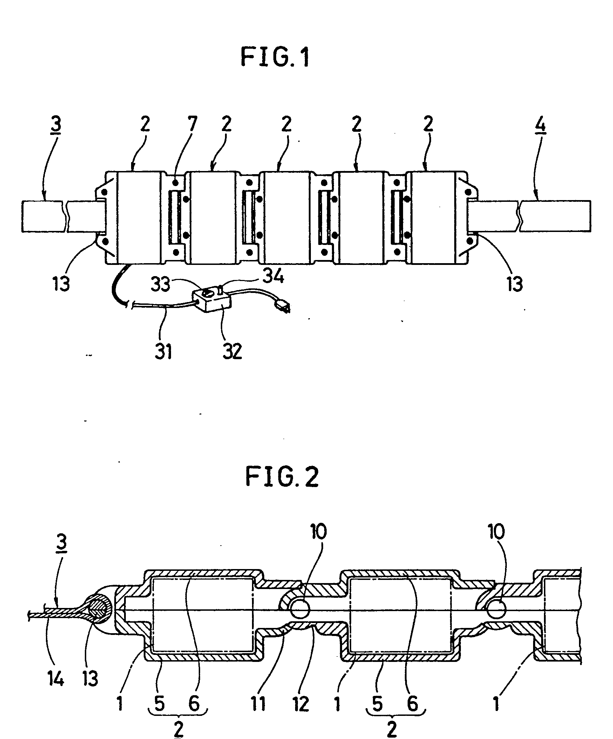

In the therapeutic apparatus for generating a magnetic field as shown in Figs. 1 through 4, magnetic field generators 1 are housed and disposed inside a plural number of cases 2 respectively and the cases are pivotally coupled together in a line. Connecting members 3 and 4 capable of mutual connection are mounted on the cases 2 and 2 located at both ends.

Each case 2 is formed by mating open end faces of a pair of coupling members 5 and 6 fixed together at a plural number of positions by screws 7. Protrusions 8 are provided at the corners of the open end face of a respective one of the coupling members 5 and 6 and grooves are provided on the face of the other member (not illustrated). When the coupling members 5 and 6 are united one with another, the protrusions 8 are engaged with the grooves.

Protruding axles 9 and 9 and the holes 10 and 10 which receive the axles of a neighbouring case are formed in the upward and downward directions on the side end face of each case 2. The protruding axles 9 thus engage with the receiving holes 10 between the adjacent cases 2 and 2, and respective cases 2 and 2 are thereby pivotally coupled with each other in a freely rotatable fashion.

Furthermore, a contact piece 11 is formed at the coupling portion between adjacent cases 2 and 2 and can engage with a stopping groove 12 on the protruding axle side to limit rotation between the cases.

On the cases 2 and 2 located at both ends, mounting axles 13 and 13 are provided at the outside end parts and belts 14 constituting the coupling members 3 and 4 are mounted on the respective mounting axles 13 and 13. Face fasteners 15 and 16 interlocking with each other are mounted at the tip of one belt 14 and along the whole length of the other belt 14 and the belts 14 and 14 are coupled by interlocking these face fasteners 15 and 16 with each other. In this way the therapeutic apparatus can be wound around and fixed to the appropriate portion of the patient's body. Furthermore, the coupling members 3 and 4 can undergo a change in design, such as providing connecting metal fittings at the tip of each belt 14 or the like, i.e. are not limited to the above-mentioned embodiment.

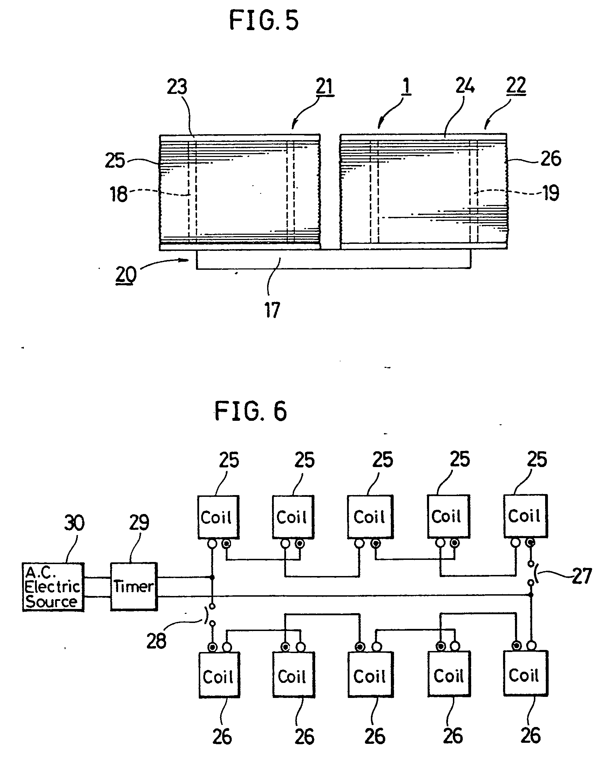

As is shown in Fig. 5 the above-mentioned magnetic field generator 1 is composed of a laminated iron core 20 provided with leg parts 18 and 19 at both ends of a base board 17 and of coil bobbins 21 and 22 engaged with respective leg parts 18 and 19. The coil bobbins 21 and 22 are formed by winding coils 25 and 26 around frames with flange 23 and 24 respectively, and are fixed to the hollow inner part of the case 2 with the tip faces of the leg parts 18 and 19 facing one of the above-mentioned coupled bodies. Coils 25 and 26 of both coil bobbins 21 and 22 are connected in a manner such that the alternating magnetic fields generated by energizing are intensified by each other, and as shown in Fig. 6, respective coils 25 and 26 in each magnetic field generator 1 are connected in series in turn. Thermostats 27 and 28 are installed in the coil connected circuit for limiting rises in temperature of the coils 25 and 26. This circuit is connected to an AC supply through a timer 29 and an arbitrary operation time is set by the timer 29. This timer 29 is, as shown in Fig. 1, housed and disposed in a control case 32 mounted on the tip of a lead-out wire 31, and a knob 33 for time setting and a power switch 34 are disposed on the upper surface of the case 32.

When using the therapeutic apparatus for generating a magnetic field, the apparatus is mounted and fixed to the affected portion of the patient's body where stiffness, pain or other ailment is felt in a manner such that the therapeutic apparatus is wound around the affected portion. Subsequently, the timer 29 is set with the knob 33, and thereby the respective coils 25 and 26 of each magnetic field generator 1 are energized and the superposed magnetic fluxes of both coils 25 and 26 act on the patient's body from the end faces of the leg parts 18 and 19 of the iron core 20. At the same time, each case 2 vibrates, and this vibration exerts a massaging effect on the body, and also a warming effect is given by the generation of Joule heat. The magnetic field generator 1 acts intensively on the affected portion in a wide range while surrounding it, thereby enhancing the circulation of the blood at the portion where stiffness or pain is felt to relieve the condition of the disease.

## Claims

1. A therapeutic apparatus for generating a magnetic field comprising a plurality of cases (2) connected in series, means (9, 10) pivotally connecting each case (2) to the preceding case (2), and a magnetic field generator (1) housed within each case, wherein the cases (2) positioned at both ends of said series of cases (2) are provided with respective connecting members (3, 4) capable of being mutually connected together, characterized in that adjacent cases are pivotally connected together by means of protruding axles (9) and cooperating receiving holes (10) for receiving said protruding axles (9), with said protruding axles and said receiving holes (10) being provided on and in said cases (2) respectively; and in that said magnetic field generators (1) generate an alternating magnetic field by conducting an alternating current through coil means (25, 26) provided on an iron core (20).

2. A therapeutic apparatus in accordance with claim 1, characterized in that said protruding axle means comprises a pair of protruding axles (9) formed at one side of one case (2) and engaging in a pair of receiving holes (10) formed at the adjacent side of the adjacent case (2).

3. A therapeutic apparatus in accordance with claim 1 or claim 2, characterized in that each case (2) comprises first and second shells (5, 6) shaped to receive a respective magnetic field generator (1) between them, with said shells being preferably secured together by screws (7).

4. A therapeutic apparatus in accordance with claims 2 and 3, characterized in that one of said shells (5) is provided with a contact piece (11) adjacent said receiving holes (10); and in that said contact piece (11) is capable of abutting against structure (12) on the next adjacent case (2) to limit the extent of relative pivotal movement of adjacent cases (2) relative to each other.

## Patentansprüche

1. Therapeutische Vorrichtung zur Erzeugung eines Magnetfeldes, mit einer Vielzahl von in einer Reihe verbundenen Gehäusen (2), schwenkbar jedes Gehäuse (2) mit vorhergehenden Gehäuse (2) verbindenden Mitteln (9, 10) und in jedem Gehäuse einem darin aufgenommenen Magnetfeld-Generator (1), wobei die an beiden Enden der Reihe von Gehäusen (1) befindlichen Gehäuse (2) mit jeweiligen gegenseitig miteinander verbindbaren Verbindungsgliedern (3, 4) versehen sind, dadurch gekennzeichnet, daß benachbarte Gehäuse mittels vorstehender Achsen (9) und damit zusammenwirkender Aufnahme-Öffnungen zur Aufnahme der vorstehenden Achsen (9) schwenkbar miteinander verbunden sind, wobei die vorstehenden Achsen und die Aufnahme-Öffnungen jeweils an bzw. in den Gehäusen (2) vorgesehen sind; und daß die Magnetfeld-Generatoren (1) ein Wechsel-Magnetfeld erzeugen, indem sie einen Wechselstrom durch an einem Eisenkern (20) vorgesehene Spulenmittel (25, 26) leiten.

2. Therapeutische Vorrichtung nach Anspruch 1, dadurch gekennzeichnet, daß die vorstehenden Achsenmittel ein vorstehendes Achsenpaar (9) umfassen, das an einer Seite eines Gehäuses (2) gebildet ist und mit einem Paar an der benachbarten Seite des benachbarten Gehäuses ausgebildeten Aufnahme-Öffnungen (10) in Eingriff steht.

3. Therapeutische Vorrichtung nach Anspruch 1 oder 2, dadurch gekennzeichnet, daß jedes Gehäuse (2) erste und zweite Mäntel (5, 6) umfaßt, die zur Aufnahme eines jeweiligen Magnetfeld-Generators zwischen einander geformt sind, wobei die Mäntel vorzugsweise durch Schrauben (7) aneinander befestigt sind.

4. Therapeutische Vorrichtung nach Ansprüchen 2 und 3, dadurch gekennzeichnet, daß einer (5) der Mäntel mit einem Kontaktstück (11) in der Nähe der der Aufnahme-Öffnungen versehen ist und daß das Kontaktstück (11) befähigt ist, gegen Strukturen (12) an dem nächstbenachbarten Gehäuse (2) anzuschlagen, um das Ausmaß der relativen Schwenkbewegung benachbarter Gehäuse (2) relativ zueinander zu begrenzen.

## Revendications

1. Appareil thérapeutique pour générer un champ magnétique, comprenant plusieurs boîtiers (2) connectés en série, des moyens (9, 10) articulant chaque boîtier (2) au boîtier précédent (2), et un générateur (1) de champ magnétique logé à l'intérieur de chaque boîtier, les boîtiers (2) situés aux deux extrémités de ladite série de boîtiers (2) comportant des éléments respectifs (3, 4) de connexion pouvant être connectés mutuellement entre eux, caractérisé en ce que des boîtiers adjacents sont articulés entre eux au moyen d'axes (9) en saillie et de trous (10) de réception coopérants destinés à recevoir lesdits axes (9) en saillie, lesdits axes en saillie et lesdits trous (10) de réception étant prévus sur et dans lesdits boîtiers (2), respectivement; et en ce que lesdits générateurs (1) de champ magnétique génèrent un champ magnétique alternatif par conduction d'un courant alternatif à travers des moyens à bobines (25, 26) disposés sur un noyau de fer (20).

2. Appareil thérapeutique selon la revendication 1, caractérisé en ce que lesdits moyens à axes en saillie comprennent deux axes (9) en saillie formés sur un premier côté d'un premier boîtier (2) et s'engageant dans deux trous de réception (10) formés dans le côté adjacent du boîtier adjacent (2).

3. Appareil thérapeutique selon la revendication 1 ou la revendication 2, caractérisé en ce que chaque boîtier (2) comprend des première et seconde coques (5, 6) configurées de façon à recevoir entre elles en générateur respectif (1) de champ magnétique, lesdites coques étant avantageusement fixées l'une à l'autre par des vis (7).

4. Appareil thérapeutique selon les revendications 2 et 3, caractérisé en ce que l'une desdites coques (5) comporte une pièce (11) de contact adjacente auxdits trous (10) de réception; et en ce que ladite pièce (11) de contact peut entrer en butée contre une structure (12) située sur le boîtier (2) immédiatement adjacent afin de limiter l'amplitude du mouvement relatif d'articulation des boîtiers adjacents (2) l'un par rapport à l'autre.

# FIG.1

# FIG.2

## FIG. 3

## FIG. 4

# FIG. 5

# FIG. 6